# EUROPEAN PATENT SPECIFICATION

(11) **EP 2 941 121 B1**
(45) Date of publication and mention of the grant of the patent: **21.11.2018**
(21) Application number: 14735343.7
(22) Date of filing: 06.01.2014
(51) Int. Cl.: A01K 1/00, A61B 5/00, A01K 15/02, A61B 5/0205, A61B 5/024, A61B 5/0478, A61B 5/0484, A61B 5/16, A61B 5/11

(54) **BIOLOGICAL SENSOR BASED SYSTEM FOR DETECTING MATERIALS**
SYSTEM AUF BASIS EINES BIOLOGISCHEN SENSORS ZUR ERKENNUNG VON MATERIALIEN
SYSTÈME BASÉ SUR UN CAPTEUR BIOLOGIQUE POUR DÉTECTER DES MATÉRIAUX

(30) Priority: 07.01.2013 IL 22412613
(43) Date of publication of application: 11.11.2015
(73) Proprietor: Biocube Diagnostics Ltd., 4522166 Hod Hasharon (IL)
(72) Inventor: NIR, Golan, 4522166 Hod Hasharon (IL); GOREN, Tamir, 7177864 Modi'in (IL)
(74) Representative: J A Kemp
(86) International application number: PCT/IL2014/050014
(87) International publication number: WO 2014/106852

(56) References cited:
- EP-A1- 1 942 342
- WO-A2-2012/100081
- US-A1- 2008 236 514
- US-A1- 2012 077 159
- US-A1- 2012 103 060
- US-A1- 2012 103 060

## Description

### Field of the Invention

The present invention relates to the field of detecting target materials. More particularly, the invention relates to a biological sensor based system for detecting odors emitted from target materials, such as dangerous materials, illegal materials or from the body of a subject (human or animal) who may have diseases (such as cancer), and for automatic providing alerts regarding any change in the functionality of sensors upon detecting a predefined target odor.

### Background of the Invention

It is well known to use a biological sensor, and more specifically, an animal, to detect dangerous or illegal materials, chemical materials and biological materials by the odors emitted therefrom. Also, odors emitted from the body of a living subject (human or animal) may provide indications about the physiological or mental state of a subject, such as early stages of cancer, an incubation stage of an epidemic etc. Therefore, fast and reliable detection of such emitted odors is highly desirable. However, biological sensors have physical limitations and therefore can perform only short term detection operations. With respect to a dog, for example, law enforcement agencies limit the consecutive working time during a detection operation to intervals of up to 20 minutes due to fatigue and a lack of attention span beyond this time and the total working time during an 8 hour shift is 60 minutes in a comfort working environment.

During prior art odor detection methods whereby the dog walks or runs back and forth between different stations in order to detect a target odor, the dog suffers from a significant loss of energy as a result of its physical activity. The dog consequently becomes tired and pants, causing moisture on the tongue to evaporate and to cool the tongue so that the blood flow through the tongue and the respiratory system will also becomes cooled. When a dog pants, however, the dog is forced to open its mouth and a reduced amount of air flows over its nose, results in a less effective odor detection operation. The tiredness of a dog becomes exacerbated during a hot day or when located in hot surroundings, such as within a closed car. Consequently, the duration of an odor detection operation for trained dogs is generally limited.

US 7,921,810 discloses a method for detecting a target substance by training an animal to execute a particular avoidance action upon detecting a target substance in order to avoid an aversive stimulus such as an electric shock, a loud noise, and a bright light. In an operational phase, the trained animal is exposed to air flowing through a container containing a specimen odor to be examined. Sensors produce a useful output signal when the trained animal performs the avoidance action. An arousal stimulus generator is periodically actuated to arouse the animal and to assure that the animal will be awake for detecting the target odor. The animal is housed within a cage that includes the basic living necessities for the animal during the training phase and the operational phase.

By being subjected to an aversive stimulus, the trained animal is caused to be under constant psychological stress due to the concern that it will again be subjected to an irritating negative reinforcement. As a result of the psychological stress to which the animal is subjected, the animal tends to be less cooperative and will not be able to reliably detect a target substance, leading to detection errors.

US 2012/103060 A1 discloses a system according to the pre-characterizing portion of claim 1. It describes a target odor detecting apparatus, which includes a rotatable door assembly for screening odor emitters for target odors. A screening zone is defined within the rotatable door assembly, and air is moved to an observation room for screening by an animate odor detector to detect one or more target odors. In one embodiment, first and second animate odor detectors are located in the same observation room, and in another embodiment a trainer accompanies the animate odor detector in the same observation room.

US 2012/077159 A1 discloses an apparatus and method for assessment of cognitive impairment and training of an animal subject. The subject is placed in a testing chamber, which is divided into a stimuli chamber and a subject chamber, wherein the stimuli chamber is configured to receive a trial assembly. The trial assembly includes at least one stimuli and a reward, where a target stimuli of the at least one stimuli is associated with the reward. After the subject is allowed access to the stimuli and has made a correct response, the subject is allowed to access a reward associated with the correct response. During a cognitive assessment and training session, a plurality of trials is performed such that a difficulty level is advanced when a learning criterion is reached. At least one cognitive assessment score is generated based on the plurality of trials and the at least one cognitive assessment and training session.

It is an object of the present invention to provide a biological sensor based system for detecting target substances by means of the odors emitted therefrom.

It is an additional object of the present invention to provide a biological sensor based detection system for reliably detecting a target substance.

It is an additional object of the present invention to provide a biological sensor based detection system by which the working time of the biological sensor for reliably detecting a target substance is dramatically increased with respect to the prior art.

Other objects and advantages of the invention will become apparent as the description proceeds.

### Summary of the Invention

According to the present invention there is provided the biological sensor based system of claim 1. Further aspects of the invention are set out in the dependent claims.

The present invention provides a biological sensor based system for detecting odors, comprising a compartment for completely or partially secluding and enclosing a biological sensor located therewithin and is configured to enable the biological sensor to perform normal animal motions as well as an odor detection enclosing a biological sensor located therewithin and is configured to enable the biological sensor to perform normal animal motions as well as an odor detection operation; an air inlet within the compartment through which odor laden air is introducible into an interior of the compartment; an air outlet through which the odor laden air is dischargeable from the compartment; a button (that may be depressible by an animal) which is movably connected to a wall of the compartment and is depressible by the biological sensor upon detection that the introduced odor is a predetermined target odor; a force sensor operatively connected to the animal depressible button for sensing the pressing parameters applied by the biological sensor onto the pushbutton; and an analysis unit for determining whether the pressing parameters are within an expected range for the biological sensor and for transmitting an alert signal to a law enforcement entity orrelevant official authorities (such as medical authorities) when the pressing parameters are within the expected range and the pressing parameters are indicative of a known reaction of the biological sensor after detecting the predetermined target odor.

The present invention is also directed to a biological sensor based system for detecting odors, comprising a carrier which is worn on the head and/or the body of a biological sensor and is provided with a plurality of electrode type sensors for determining electrical activity in the brain by means of an electroencephalogram (EEG), a predetermined wave pattern being indicative of a biological sensor related mental state of satisfaction when a predetermined target odor has been detected; an EEG receiver connected to the electrode type sensors for receiving generated EEG parameters; and an analysis unit for receiving the generated EEG parameters, for determining whether the generated EEG parameters are within an expected range of EEG parameters, and for transmitting an alert signal to a law enforcement entity or relevant official authorities (such as medical authorities) when the generated EEG parameters are within the expected range and the generated EEG parameters are indicative of a known reaction of the biological sensor after detecting the predetermined target odor.

The system may further comprise one or more additional signal generating sensors for sensing a corresponding physical reaction of the biological sensor during the odor detecting operation, wherein the analysis unit is operable to disregard results of the odor detecting operation when a corresponding signal generated by the one or more additional sensors is representative of a physical reaction that is inconsistent with the known reaction of the biological sensor after detecting the predetermined target odor.

The system may also comprise a video camera for capturing a body motion of the biological sensor.

The biological sensor may be a dog, and one of the additional sensors is a video camera for capturing a body motion of the dog.

The system may further comprise means for capturing the known reaction of approaching a bowl located within the compartment in search for its reward immediately following detection of the predetermined odor and after the pushbutton has been pressed.

One of the additional sensors may be a heart rate sensor for determining the stamina and awareness level of the biological sensor and whether it is presently sufficiently fit to continue performing additional odor detection operations.

The biological sensor(s) may be a dog, rodents, pigs, birds, winged creatures, amphibians, insects or a mammal, trained to depress the pushbutton with its nose or leg upon detecting a predetermined target odor.

The parameters of pressing the depressible button may be force, duration, angle or speed.

The additional signal generating sensors may be:
- a microphone installed on the animal's body or within the compartment
- a pushbutton;
- a heart rate sensor;
- a camera;
- a motion sensor;
- EEG sensor.

The system may further comprise means for generating a dominant and/or secondary encouragement when a target odor is identified.

The secondary encouragement may be:
- a distinctive sound;
- a high pitched sound;
- a clicking sound;
- voice emitted from a loudspeaker that is installed in the compartment;
- a pleasant light.

The pressing parameters may be:
- magnitude of applied force;
- duration;
- angle;
- speed.

The dominant encouragement can be any reward that causes joy to the biological sensor, such as favorite food or a favorite game.

One of the additional sensors may be an audio capturing device for capturing the audible sound produced by the biological sensor, upon detecting a predetermined target odor.

### Brief Description of the Drawings

In the drawings:
- Fig. 1 is a schematic illustration of a biological sensor based system, according to one embodiment of the present invention;

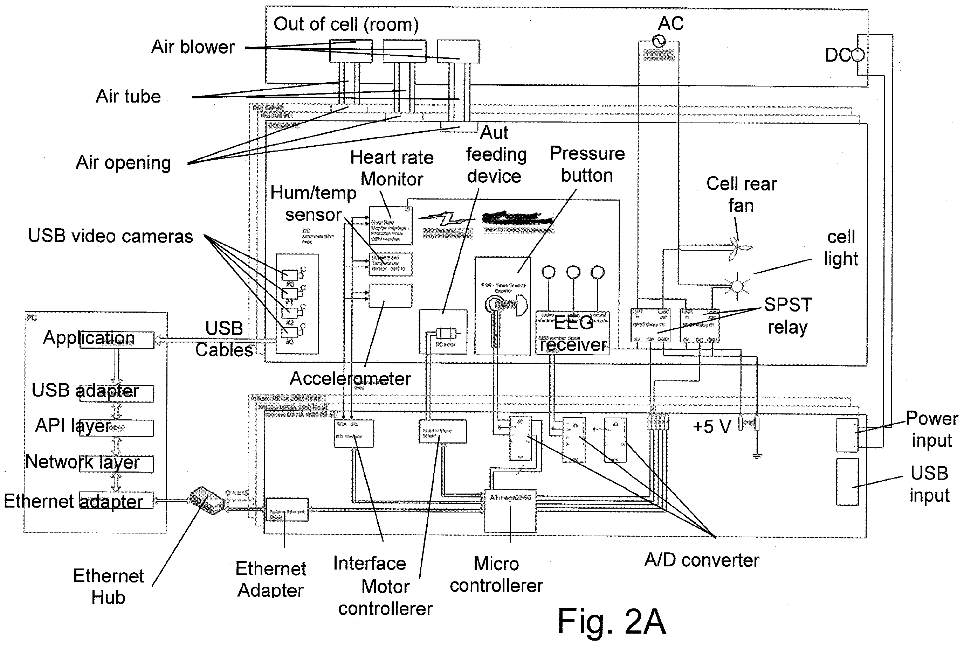

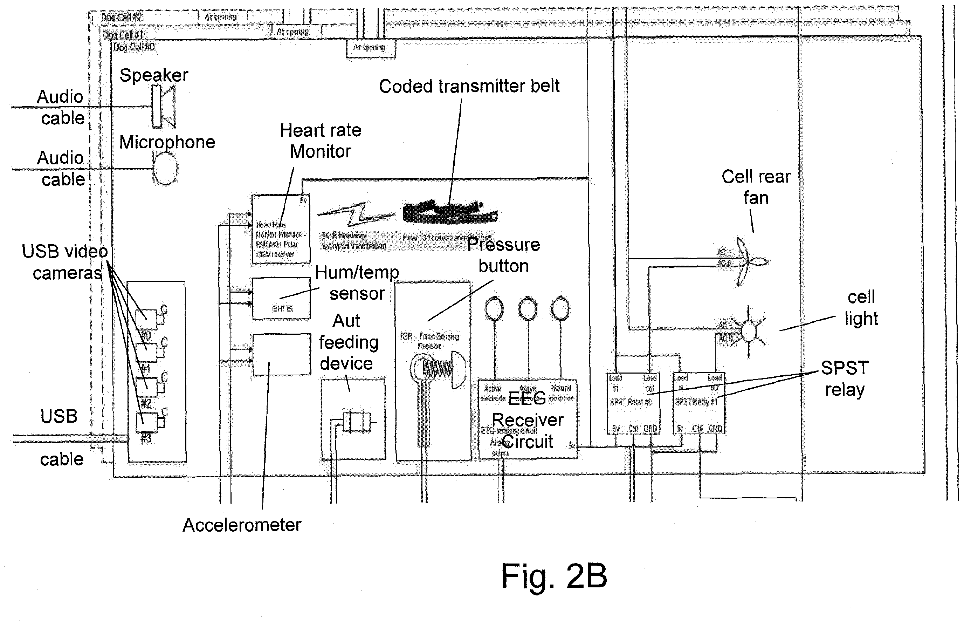

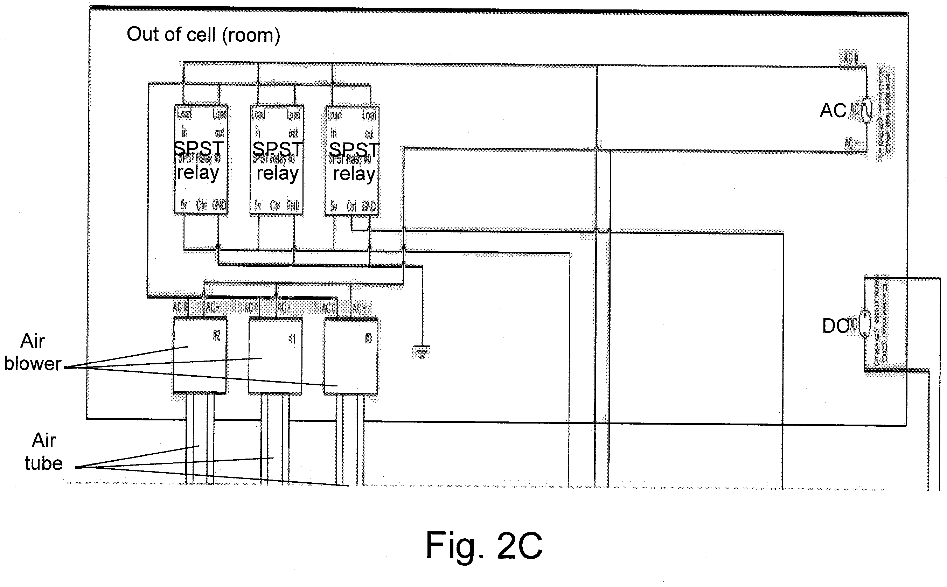

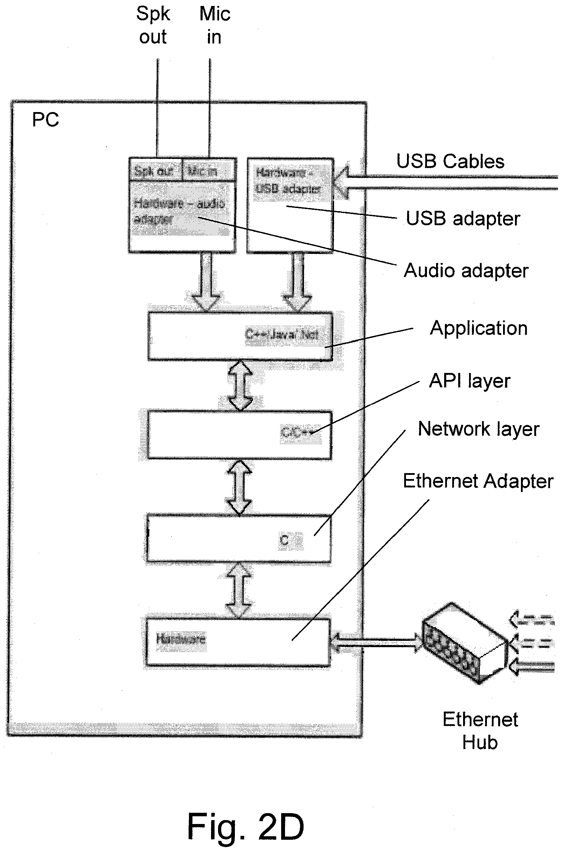

- Figs. 2a-d are an exemplary layout of the system of Fig. 1;
- Fig. 3 schematically illustrates an animal depressible pushbutton, according to one embodiment of the present invention;
- Fig. 4 schematically illustrates an animal depressible pushbutton, according to another embodiment of the present invention; and
- Fig. 5 is a schematic illustration of a biological sensor based system, according to an embodiment not in the scope of the present invention.

### Detailed Description of Preferred Embodiments

The present invention is a novel biological sensor based detection system for detecting odors emitted from dangerous or illegal materials. The biological sensor, as well as one or more signal generating sensors, is positioned within a fully or partially confined space. The signal generating sensors are adapted to measure physical reactions of the biological sensor in response to the injection within the confined space of a stream of air laden with a target odor, and to transmit the generated signals to a processor. The processor is able to determine whether the measured physical reactions are characteristic of known reactions of the biological sensor when exposed to odors emitted from a predetermined material, and to output a signal that is indicative that the predetermined material has been detected.

The following description relates to a canine biological sensor, but it should be indicated that the detection system is also reliably operable in conjunction with other types of biological sensors such as rodents, pigs birds, mammals, winged creatures, amphibians and insects.

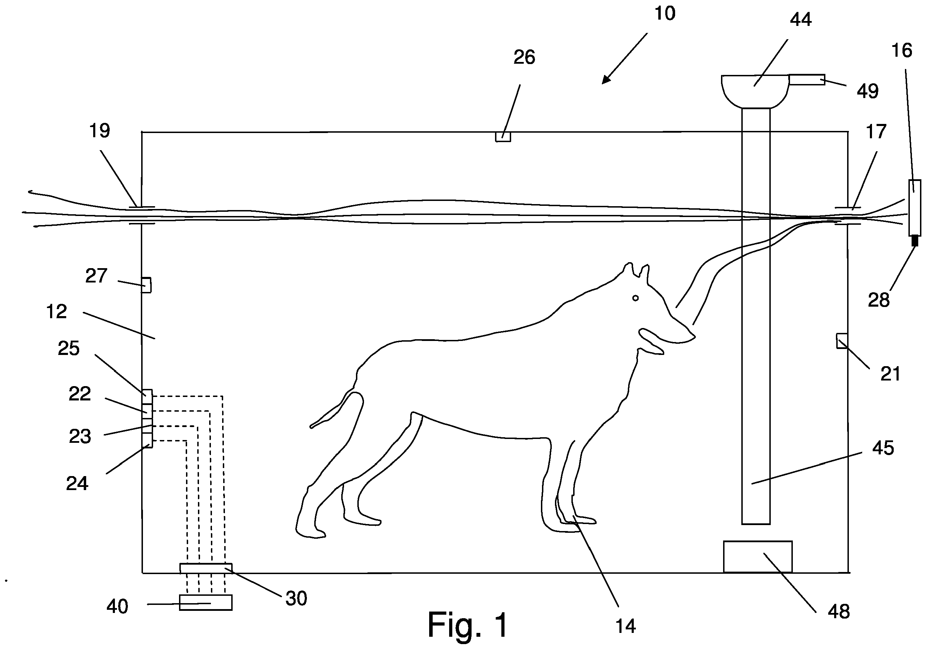

Fig. 1 schematically illustrates a system generally indicated by numeral 10, according to one embodiment of the present invention. System 10 comprises a confined space 12, referred hereinafter as a "compartment", within which a trained dog 14, which may also be referred to as the "subject dog" or the "subject animal", is retained, a blower 16 for causing an air stream to circulate through a suspected object and into compartment 12 via air inlet 17 and then via air outlet 19, a plurality of signal generating sensors 21-24 mounted within compartment 12 or on the body of subject dog 14, light source 26, a communication unit 30 for receiving the generated signals, an analysis unit 40 for analyzing the received signals, a food dischargeable member 45 extending downwardly into the interior of the compartment 12, and a bowl 48 for receiving a dominant encouragement discharged from food dischargeable member 45 without any visible human intervention. Any signal generating sensor is independent and has its own relevant parameters, which are considered while the analysis unit 40 processes the data collected from all signal generating sensors in real-time, in order to issue alerts whenever appropriate.

One or more components of system 10 may be automatically controlled according to a predetermined sequence.

An important aspect of the present invention is the ability of subject dog 14 to perform detection operations within compartment 12. As opposed to prior art canine based detection methods whereby dogs that roam freely or are leashed are subjected to target odors and consequently become tired due to the physical activity involved in the detection operation, and the duration of the working time for trained dogs in conjunction with prior art detection systems is generally limited to 20 minutes, a subject dog 14 retained within compartment 12 minimizes its physical activity. By virtue of the reduced physical activity within compartment 12 during a detection operation, subject dog 14 experiences less fatigue and an increased attention span, and is therefore able to work consecutively for at least an hour, and even more than 3 hours consecutively, while being able to accurately detect target odors.

The interior of a fully assembled compartment is sufficiently large so that the subject dog will be completely enclosed therewithin and will be able to freely perform normal canine motions such as sitting, lying down, eating, and barking, yet is sufficiently small to prevent the subject dog from running or roaming within the compartment, in order to conserve its energy.

Compartment 12 may be stationary or alternatively may be movable, such as provided with wheels or sufficiently portable so as to be transportable within a motor vehicle.

Prior to being able to accurately and reliably performing detection operations within compartment 12, subject dog 14 is trained such that it is willing to enter the confines of compartment 12. Without being properly trained, a dog forced to enter a confined space will be very irritable, moving repeatedly back and forth within its interior, and will be invariably uncooperative in performing any task, even if commanded by its handler.

Copending international application No. PCT/IL2013/050859 by the same Applicants teaches the method for training the subject dog to willingly enter compartment 12 by rewarding it only within the interior of the compartment for successfully performing certain tasks. The subject dog therefore associates pleasant memories with the compartment, being a favorite place for receiving treats such as salami and bones or enjoyable games. After a while, the subject dog will enter compartment 12 voluntarily and will perform one or more trained detection related activities, as will be described hereinafter.

The handler first trains the dog to identify a target odor by introducing the target odor into an odor emittable package, and rewarding the dog with a dominant encouragement every time that it places its nose on the package containing the target odor. The subject dog is also trained, prior to being capable of performing an odor detecting operation within the compartment, to discriminate between positive and neutral odors.

Subject dog 14 is trained to work alone within compartment 12 without being able to observe its handler, so as not to be distracted during a detection operation.

Signal generating sensor 21 is a pushbutton, e.g. positioned 40 cm above the compartment floor, for indicating that the predetermined target odor has been detected. Subject dog 14 is trained to depress the pushbutton with its nose, or by any other bodily part, by a force within a predetermined range, usually a deliberate small magnitude force, when it detects a predetermined target odor. Other pressing parameters such as its duration, angle and speed may be used to characterize the pressing type. The pushbutton may be distinctive to the dog, such as being red while the other sensors are grey, or alternatively, may be coplanar with a compartment wall while the other sensors are recessed therefrom. In addition to the pushbutton, sensor 21 comprises a force sensor for sensing the magnitude of the force applied by the dog's nose onto the pushbutton. Alternatively, the dog can press the pushbutton by his leg.

During a calibration stage and after the subject dog is properly trained to detect a predetermined target odor and to depress the pushbutton upon detecting the target odor, several detection operations are performed by the subject dog during each of which an air stream of the predetermined target odor is injected into the compartment. The magnitude of the force applied onto the pushbutton is measured for each operation. After a sufficient number of detection operations have been performed, an expected force application range for the subject dog taking into account acceptable testing and performance deviations is established and stored in analysis unit 40.

During a testing stage, the subject dog performs a plurality of detection operations to determine whether one of many suspected objects contains dangerous or illegal materials. An air stream is caused to flow through a suspected object and then be injected into compartment 12 for each detection operation. If the air stream carries particles of a dangerous or illegal material, the subject dog will detect the predetermined target odor by its olfactory sense when the concentration of the airborne material is above a threshold value and will press the pushbutton with its nose, or with any other bodily part it was trained to use, within a predetermined period of time following injection of the air stream, as a dominant indication that the predetermined target odor has been detected. After the subject dog applies a force, the signal generated by the force sensor is received by communication unit 30 and then transmitted to the processor of analysis unit 40. Analysis unit 40 will issue an alert signal to a law enforcement group when the applied force is within the expected range and the generated signal is indicative that the predetermined odor has been detected.

The subject dog is immediately rewarded for detecting the predetermined target odor, generally by a dominant encouragement, such as salami, a bone, and a game introduced to bowl 48, for increasing the subject dog's physical pleasure and serving as an incentive for accurately performing future detection operations.

At times, the subject dog may first receive a secondary encouragement when a target odor is identified. The secondary encouragement or a negative feedback may be a distinctive sound such as a high pitched or pleasant sound produced by a whistle or a clicking sound, a loudspeaker that is installed in the compartment, a visual encouragement such as pleasant light, or any other type of encouragement. The secondary encouragement provides indication to the subject dog that it performed up to expectations while detecting a target odor. After being provided with the secondary encouragement, the subject dog waits in anticipation for the dominant encouragement. The timing between receiving the secondary encouragement and dominant encouragement may be controlled, in order to improve the subject dog's performance.

The subject dog will at times trigger a false alarm when the force applied to the pushbutton is unintentionally within the expected range, for example when tired. To minimize the number of false alarms, analysis unit 40 receives secondary indications from one or more other signal generating sensors. Analysis unit 40 will disregard the signal generated by the force sensor when one or more of the secondary indications is representative of a physical reaction that is inconsistent with a known physical reaction of the subject dog after detecting a predetermined target odor.

One of the secondary indications may be a number of nose pressing actions performed by the subject dog. When the subject dog is alert and is optimally performing the detection operations, the pushbutton will be pressed only once upon detection of the predetermined target odor, as trained. If the subject dog is irritable, however, the subject dog has difficulty in controlling its detection related activities and tends to perform a plurality of nose pressing actions. Thus analysis unit 40 will disregard the dominant indication provided by a generated signal of an expected magnitude range when it is accompanied by two other signals generated by sensor 21 within a predetermined period of time since the subject dog is known not to press the pushbutton three times after detecting the predetermined target odor.

Alternatively, a secondary indication may be the duration of a pressing action performed by the subject dog. Analysis unit 40 will disregard the dominant indication provided by a generated signal of an expected magnitude range when it is characterized by a pressing duration less than a predetermined pressing duration range.

An alertly performing subject dog at times may press the pushbutton twice upon detection of the predetermined target odor when it is overexcited, in anticipation of the expected reward. Since the generation of two signals by the force sensor will not conclusively indicate that the predetermined odor has been detected, system 10 may comprise additional signal generating sensors to provide a third indication, or any other number of indications, to assist analysis unit 40 in further filtering out false alarms. An additional signal generating sensor may be a microphone installed on the animal's body or within the compartment, in order to provide indications (such as sniffing rate of the animal, barking or yowling that a trained dog may generate when detecting the target odor) to the analysis unit 40. These indications may provide information regarding the awareness level of the animal.

A third indication may be provided by sensor 24, which is a video camera for capturing the body motion of the subject dog. The Applicants have discovered that the subject dog approaches bowl 48 in search for its reward immediately following detection of the predetermined odor and after the pushbutton has been pressed. Thus a signal generated by the force detector will be filtered out by analysis unit 40 if the subject dog did not approach the bowl, knowing that the predetermined odor was not detected.

Another signal generating sensor for providing a third indication may include a heart rate sensor 22 for determining the level of the subject dog's stamina and whether it is presently sufficiently fit to continue performing odor detection operations. Heart rate sensor 22 may be mounted on the subject dog's body. The generated signal may be transmitted wirelessly or by a wired connection to communication unit 30 and then to analysis unit 40, or directly to analysis unit 40. This third indication helps to set a work schedule for the subject dog- when it has to take a break in order to be sufficiently revitalized to perform an additional odor detecting operation and what is an ideal heat rate for commencing an odor detecting operation within compartment 12. Analysis of the acquired heart rate data helps to conserve the subject dog's energy within the compartment and to allow the subject dog to release its energy outside the confines of the compartment.

Also, a motion sensor 23 may be used to determine the rate of the subject dog's movement, as another third indication of whether it is sufficiently alert during an odor detection operation. This sensor monitors the motions of the dog and can analyze his motions upon detecting a target odor.

The subject dog (or another biological sensor) can additionally be trained to produce an audible sound, upon detecting a predetermined target odor. In this case, an audio capturing device (e.g., a recorder) may be used for capturing the audible sound that is produced by the biological sensor, upon detecting the target odor. The captured audio signals can be analyzed for their auditory features, such as length, strength and harmonic structure, and can be compared to known auditory features, in order to extract characteristic features and patterns for each target odor.

After communication unit 30 receives the inputs from all types of sensors, analysis unit 40 receives the corresponding signals from the communication unit and determines according to predetermined stored rules whether the physiological and instinctively trained and un-trained (EEG) actions performed by the subject dog during the course of the odor detection operation are indicative that a target odor was accurately and conclusively detected. Analysis unit 40 then transmits an alert signal to a law enforcement agency.

Compartment 12 may also be equipped with other equipment.

A temperature/humidity sensor 25 for detecting the temperature and/or humidity within compartment 12 may also be provided, in order to monitor the comfort level of the subject dog. For example, air conditioned cool air may be introduced into the compartment via a designated opening on a hot day. Sensor 23 may be mounted on a compartment wall. Alternatively, sensor 23 may be positioned on the body of the subject dog, such as on a collar or by means of a harness, in order to monitor the body temperature.

A controllable light source 26 may be mounted on the ceiling 15 of compartment 12 above the subject dog's head and body, or on any other region within the compartment, in such a way so as not to distract or interfere with the subject dog during an odor detection operation. Light source 26 is preferably one that generates minimal thermal heating, for increased comfort to the subject dog.

The subject dog may be trained such that illumination of light source 26, after entering into a dark compartment, indicates commencement of the odor training operation. Similarly, deactivation of light source 26 indicates the end of the odor training operation. Light source 26 may be manually activated and deactivated by the handler, or may be automatically activated and deactivated by the processor of analysis unit 40 in response to a predetermined timing sequence.

Light source 26 may provide the light for a secondary encouragement when a target odor has been detected, or may provide unpleasant light as a reprimand when the subject dog performed inaccurately.

A sound emitter 27 mounted on a wall of the compartment may provide an audible secondary encouragement when a target odor has been detected, or may emit an unpleasant sound such as a loud and strident sound or a sound at a frequency unpleasant to the subject dog, as a reprimand when it performed inaccurately. An emitted sound may also serve as communication between the handler and the subject dog for various purposes, for example to stimulate the subject dog if it exhibiting a lack of concentration or to command it to perform a certain action by a whistle or a clicking sound.

A switch 28 or any other suitable means for manually activating or deactivating one or more blowers 16 for directing an air stream into the compartment may also be provided. Each blower 16 may also be automatically and individually controlled by the processor of analysis unit 40. Switch 28 may also be configured to control lighting means, fans or any other desired control device.

Automatic dispenser 49 is positioned externally from compartment 12 and is commanded by the processor of analysis unit 40 to dispense a predetermined dominant encouragement at a predetermined time following transmission of the alert signal. The dispensed dominant encouragement is introduced into an upper enlarged food introducible chamber 44 in communication with, and located above, food dischargeable member 45, e.g. a pipe, extending downwardly into the interior of compartment 12. The bottom of pipe 42 may be positioned above, e.g. 10 cm, floor 37 of compartment 12. Bowl 48 is adapted to receive food, or any other dominant encouragement, delivered through member 45. The subject dog is therefore able to receive its dominant encouragement directly within compartment 12 immediately after accurately detecting the target odor and performing the desired action, and will be willing to perform additional odor detection operations.

Food dischargeable member 45 and automatic dispenser 49 may be miniaturized, folded or otherwise provided in a compact condition, so as to be carried by a subject dog performing odor detecting operations outside of a compartment.

Figs, 2A-C illustrate an exemplary layout of system 10.

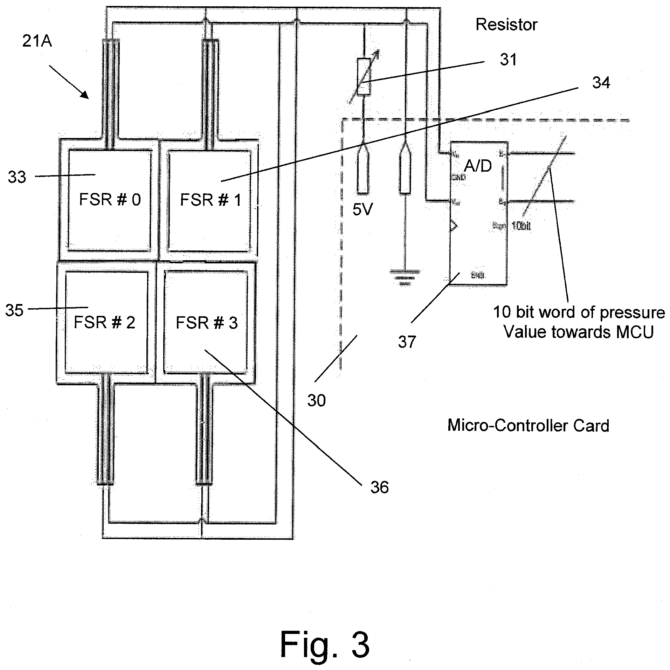

As shown in Fig. 3, animal depressible pushbutton 21A may comprise a planar surface 33 to which the subject dog applies a force during an odor detection operation and a force sensing resistor 31 that changes its resistance in response to the force applied on surface 33. An exemplary force sensing resistor is the FSR 406 manufactured by Interlink Electronics capable of sensing an applied force ranging from 10 g to 10 kg and having a surface area of 19.4 cm². Pushbutton 21A may also comprise a plurality of adjacently positioned surfaces 33-36, or alternatively a single enlarged surface. Compressed foam may be adhesively attached to the plurality of surfaces 33-36, to serve as a single pushbutton. A/D converter 37 of communication unit 30 receives the voltage applied by each of the resistors 31 in parallel, a higher voltage being received by the microcontroller when a lower force magnitude is applied.

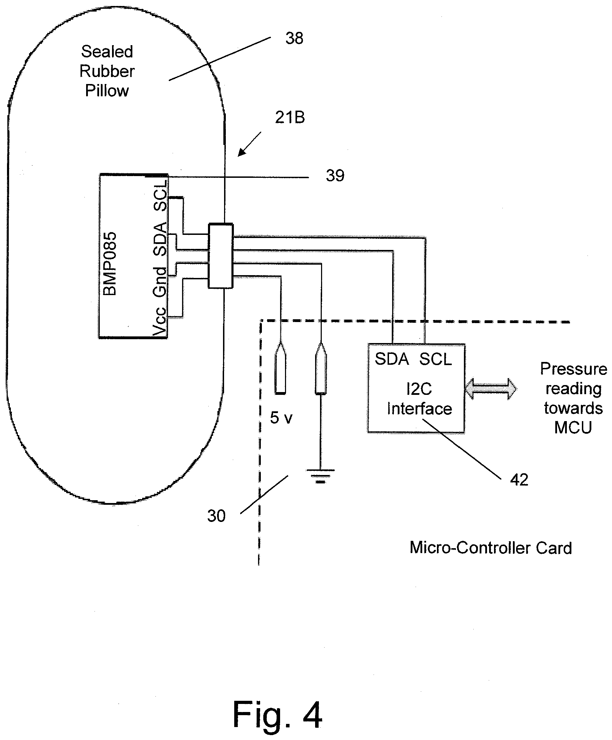

Alternatively, as shown in Fig. 4, animal depressible pushbutton 21B may be embodied by an opaque airbag 38, e.g. made of rubber, in which is housed a pressure sensor 39. The pressure reading detected by pressure sensor 39 will change when the subject dog applies a force to airbag 38, allowing a magnitude of the applied force to be determined. The signal generated by pressure sensor 39 is delivered to bus 42 for facilitating communication on the same circuit board.

The size of the compartment and of the pushbutton, as well as the necessary force needed to depress the pushbutton will vary, depending on the size of the subject animal that is performing an odor detecting operation.

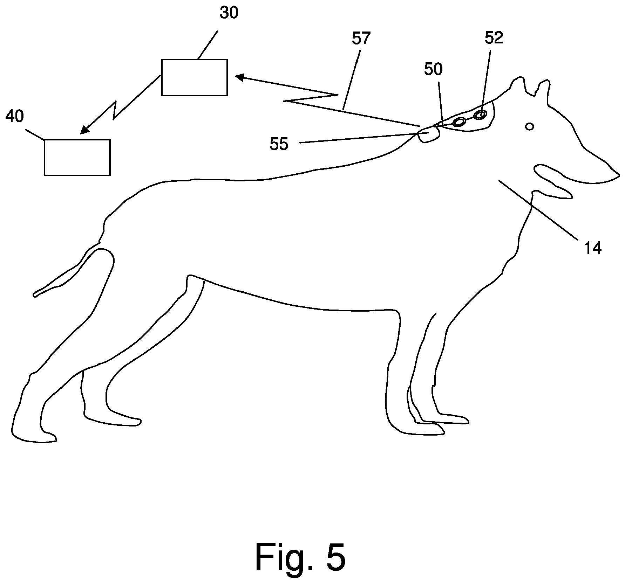

In an embodiment not in the scope of the invention schematically illustrated in Fig. 5, an animal interfaceable member 50 may be worn on the head of the subject dog 14 and be provided with a plurality of electrode type sensors 52 for determining electrical activity in the brain by means of an electroencephalogram (EEG), so that a predetermined wave pattern will be indicative of whether the predetermined target odor has been detected. Animal interfaceable member 50 may also be a harness worn on the head. Alternatively, the electrodes 52 may be attached by adhesion or by pins to the scalp of the subject dog.

It should be indicated that even though the main functionality of the EEG is to provide true detection of a target odor, it may be used to monitor the awareness and the physiologic state and the mental state (fear, stress etc.) of the biological sensor, to detect false alerts, to monitor parameters that affect its physiologic state, etc.

The electrodes 52 are connected to by a wired connection to an EEG receiver 55. EEG receiver 55, which may be part of the communication unit, provides a graphical indication regarding the amplitude of the brain waves during an odor detecting operation, as well as frequency or temporary changes of the EEG signals. The generated EEG data is transmitted to communication unit 30 via communication link 57, which may be either a wired or a wireless connection, whereupon analysis unit 40 will receive the EEG data and determine whether a target odor has been detected. Alternatively, the generated EEG data may be transmitted directly to analysis unit 40.

During a calibration stage and after the subject dog is properly trained to detect a predetermined target odor, several detection operations are performed by the subject dog during each of which an air stream of the predetermined target odor is injected into the compartment. A range of EEG parameters, including a range of change in EEG parameters, which are manifested during a positive odor detection event, i.e. when the predetermined target odor has been detected, is recorded and stored in analysis unit 40. Thus manifestation of EEG parameters during a testing stage that are within the stored range (hereinafter "expected EEG parameters") indicates that the target odor has been detected and that the subject dog has achieved a sense of satisfaction, in anticipation of the expected reward.

Generation of the expected EEG parameters may constitute a secondary indication that the predetermined target odor has been accurately detected. Other secondary or third indications described hereinabove may also be employed.

Alternatively, generation of the expected EEG parameters may constitute a dominant or secondary indication that the predetermined target odor has been accurately detected. Thus in one embodiment, the odor detection system may be provided without a pushbutton while a secondary indication of accurate target odor detection may be received from the subject dog in the form of one or more trained detection related activities such as sitting, lying down or barking. In another embodiment, the subject dog need not be retained in a compartment and may be leashed or may roam freely during an odor detection operation insofar as it is wearing animal interfaceable member 50, in order to determine whether the expected EEG parameters are generated.

The detection of odors according by a subject dog located outside of a compartment while wearing animal interfaceable member 50 provided with electrodes 52 is much more accurate than prior art odor detection methods that suffer from inaccuracies when a subject dog is hungry and irritable, since the generated EEG parameters will indicate whether a trained detection related activity is reflective of a detected target odor or of a fatigued dog that is inaccurately performing an activity in order to receive a reward. Thus the risk of false alarms can be essentially eliminated.

While some embodiments of the invention have been described by way of illustration, it will be apparent that the invention can be carried out with many modifications, variations and adaptations, and with the use of numerous equivalents or alternative solutions that are within the scope of persons skilled in the art, without exceeding the scope of the claims.

## Claims

1. A biological sensor based system (10) for detecting odors, comprising:
a) a compartment (12) for completely secluding and enclosing a biological sensor (14) located therewithin, said compartment being sufficiently large to enable said biological sensor to perform normal animal motions as well as an odor detection operation;
b) an air inlet (17) within said compartment through which odor laden air is introducible into an interior of said compartment;
c) an air outlet (19) through which said odor laden air is dischargeable from said compartment;
d) one or more signal generating sensors (21, 24, 52) for measuring physical reactions of said biological sensor in response to the introduction into said compartment interior of said odor laden air; and
e) an analysis unit (40) for determining whether said measured physical reactions are indicative of known reactions of said biological sensor upon detecting a predetermined target odor,
**characterized in that**:
said compartment is sufficiently small to ensure that there will be no space in said compartment to contain a handler or further biological sensors performing odor detection operations in addition to said biological sensor, and that said biological sensor will be prevented from running or roaming therewithin, to conserve its energy;
said analysis unit is configured to automatically transmit a first alert signal to a law enforcement entity or relevant official authorities when said measured physical reactions are found to be indicative of said known reaction of said biological sensor upon detecting said predetermined target odor;
said system further comprises:
f) one or more additional signal generating sensors (22-24) for determining an awareness level of the biological sensor and whether the biological sensor is presently sufficiently fit to continue performing said odor detection operations, and
wherein said analysis unit (40) is additionally configured to receive an awareness level indicative signal from said one or more additional signal generating sensors (22-24).

2. The system according to claim 1, further comprising:
a) an animal depressible button (21, 21A, 22B) which is movably connected to a wall of said compartment (12) and is depressible by said biological sensor (14) upon detection that said introduced odor is the predetermined target odor; and
b) a force sensor (31, 39) constituting said one or more signal generating sensors, which is operatively connected to said animal depressible button, for sensing pressing parameters applied by said biological sensor onto said button,
wherein said analysis unit (40) is adapted to determine whether said pressing parameters are within an expected range for said biological sensor and to transmit the first alert signal to the law enforcement entity or relevant official authorities when said pressing parameters are within said expected range and said pressing parameters are indicative of said known reaction of said biological sensor after detecting said predetermined target odor.

3. The system according to claim 1, further comprising:
a) a carrier (50) which is wearable on the head and/or the body of said biological sensor (14) and is provided with a plurality of electrode type sensors (52) constituting said one or more signal generating sensors for determining electrical activity in the brain by means of an electroencephalogram (EEG), a predetermined wave pattern being indicative of a biological sensor related to a mental state of satisfaction when said predetermined target odor has been detected; and
b) an EEG receiver (55) connected to said electrode type sensors for receiving generated EEG parameters,
wherein said analysis unit (40) is adapted to receive said generated EEG parameters, to determine whether said generated EEG parameters are within an expected range of EEG parameters, and to transmit said first alert signal to the law enforcement entity or relevant official authorities when said generated EEG parameters are within said expected range and said generated EEG parameters are indicative of said known reaction of said biological sensor after detecting said predetermined target odor.

4. The system according to claim 1, wherein the one or more additional signal generating sensors (22-24) are also adapted to sense a corresponding physical reaction of the biological sensor (14) during the odor detecting operation, wherein the analysis unit (40) is operable to disregard results of the odor detecting operation when a corresponding signal generated by said one or more additional sensors is representative of a physical reaction that is inconsistent with the known reaction of the biological sensor after detecting the predetermined target odor.

5. The system according to claim 1, in which the one or more additional signal generating sensors are selected from the group of:
- a microphone installed on the biological sensor's body or within the compartment (12);
- a pushbutton (21, 21A, 21B);
- an EEG sensor (52);
- a heart rate sensor (22) for determining the heart rate of the biological sensor (14);
- a video camera (24) for capturing a body motion of the biological sensor;
- an audio capturing device for capturing an audible sound produced by the biological sensor upon detecting the predetermined target odor; and
- a motion sensor (23) for capturing a body motion of the biological sensor.

6. The system according to claim 2, wherein the biological sensor is a dog (14), further comprising a video camera (24) for capturing a body motion of the dog during the odor detecting operation, wherein the analysis unit (40) is operable to disregard results of the odor detecting operation when a body motion captured by said video camera is representative of a physical reaction that is inconsistent with the known reaction of dog after detecting the predetermined target odor.

7. The system according to claim 2, further comprising means (24) for capturing the measured physical reaction of approaching a bowl (48) located within the compartment (12) in search for its reward immediately following detection of the predetermined odor and after the button (21, 21A, 22B) has been pressed, wherein the analysis unit (40) is operable to disregard results of the odor detecting operation when a body motion captured by said video camera is representative of a physical reaction that is inconsistent with the known reaction of dog after detecting the predetermined target odor.

8. The system according to claim 1, wherein the biological sensor (14) is selected from the group of:
- a dog;
- rodents;
- pigs;
- birds;
- mammals;
- winged creatures;
- amphibians; and
- insects.

9. The system according to claim 2, wherein the biological sensor (14) is a mammal, trained to depress the button (21, 21A, 22B) with its nose or leg upon detecting the predetermined target odor.

10. The system according to claim 2, wherein the pressing parameters are selected from the group consisting of:
- Force;
- Duration;
- Angle; and
- Speed.

11. The system according to claim 1, further comprising means for generating a dominant and/or secondary encouragement (26-28, 49) when the target odor is identified.

12. The system according to claim 11, wherein the dominant encouragement is selected from the group consisting of:
- a favorite food; and
- a favorite game,
wherein the secondary encouragement is selected from the group consisting of:
- a distinctive sound;
- a high pitched sound;
- a clicking sound;
- a voice emitted from a loudspeaker that is installed in the compartment; and
- a pleasant light.

13. The system according to claim 11, in which the means for generating a dominant encouragement when a target odor is identified is an automatic dispenser (49), and optionally
in which the automatic dispenser (49) comprises a food introducible chamber (44) in communication with, and located above, the compartment (12), and a food dischargeable member (45) extending downwardly into an interior of the compartment and through which said introduced food is dispensable.

14. The system according to claim 1, in which the relevant official authorities are medical authorities.

## Patentansprüche

1. Auf einem biologischen Sensor basierendes System (10) zum Detektieren von Gerüchen, umfassend:
a) eine Kammer (12) zum vollständigen Abschließen und Umschließen eines biologischen Sensors (14), der sich darin befindet, wobei die Kammer ausreichend groß ist, um dem biologischen Sensor zu ermöglichen, normale tierische Bewegungen sowie einen Geruchsdetektionsvorgang auszuführen;
b) einen Lufteinlass (17) innerhalb der Kammer, durch den geruchsbeladene Luft in ein Inneres der Kammer einführbar ist;
c) einen Luftauslass (19), durch den die geruchsbeladene Luft aus der Kammer ableitbar ist;
d) ein oder mehrere Signalerzeugungssensoren (21, 24, 52) zum Messen von physikalischen Reaktionen des biologischen Sensors als Reaktion auf das Einführen der geruchsbeladenen Luft in das Kammerinnere; und
e) eine Analyseeinheit (40) zum Bestimmen, ob die gemessenen physikalischen Reaktionen nach dem Detektieren eines vorbestimmten Zielgeruchs auf bekannte Reaktionen des biologischen Sensors hinweisen,
**dadurch gekennzeichnet, dass**:
die Kammer ausreichend klein ist, um sicherzustellen, dass es keinen Raum in der Kammer gibt, um eine Handhabevorrichtung oder weitere biologische Sensoren aufzunehmen, die zusätzlich zu dem biologischen Sensor Geruchsdetektionsvorgänge ausführen, und dass verhindert wird, dass der biologische Sensor darin läuft oder umherwandert, um seine Energie zu erhalten;
die Analyseeinheit konfiguriert ist, automatisch ein erstes Warnsignal an eine Rechtsdurchsetzungsentität oder an relevante Behörden zu senden, wenn festgestellt wird, dass die gemessenen physikalischen Reaktionen auf die bekannte Reaktion des biologischen Sensors nach dem Detektieren des vorbestimmten Zielgeruchs hinweisen;
wobei das System ferner umfasst:
f) ein oder mehrere zusätzliche Signalerzeugungssensoren (22-24) zum Bestimmen eines Bewusstseinsniveaus des biologischen Sensors und ob der biologische Sensor gegenwärtig ausreichend einsatzfähig ist, um weiterhin die Geruchsdetektionsvorgänge auszuführen, und
wobei die Analyseeinheit (40) zusätzlich konfiguriert ist, ein auf ein Bewusstseinsniveau hinweisendes Signal von dem einen oder den mehreren zusätzlichen Signalerzeugungssensoren (22-24) zu empfangen.

2. System nach Anspruch 1, ferner umfassend:
a) eine durch ein Tier drückbare Taste (21, 21A, 22B), die mit einer Wand der Kammer (12) beweglich verbunden ist und durch den biologischen Sensor (14) nach der Detektion, dass der eingeführte Geruch der vorbestimmte Zielgeruch ist, drückbar ist; und
b) einen Kraftsensor (31, 39), der den einen oder die mehreren Signalerzeugungssensoren bildet, welcher wirkend mit der durch ein Tier drückbaren Taste zum Erfassen von Drückparametern verbunden ist, die durch den biologischen Sensor auf die Taste ausgeübt werden,
wobei die Analyseeinheit (40) angepasst ist, zu bestimmen, ob die Drückparameter innerhalb eines erwarteten Bereichs für den biologischen Sensor liegen, und das erste Warnsignal an die Rechtsdurchsetzungsentität oder die relevanten Behörden zu senden, wenn die Drückparameter innerhalb des erwarteten Bereichs liegen und die Drückparameter nach dem Detektieren des vorbestimmten Zielgeruchs auf die bekannte Reaktion des biologischen Sensors hinweisen.

3. System nach Anspruch 1, ferner umfassend:
a) einen Träger (50), der am Kopf und/oder dem Körper des biologischen Sensors (14) tragbar ist und mit mehreren Elektrodensensoren (52) versehen ist, die den einen oder die mehreren Signalerzeugungssensoren zum Bestimmen von elektrischer Aktivität im Gehirn mittels eines Elektroenzephalogramms (EEG) bilden, wobei ein vorbestimmtes Wellenmuster auf einen mit einem geistigen Zufriedenheitszustand in Zusammenhang stehenden biologischen Sensor hinweist, wenn der vorbestimmte Zielgeruch detektiert wurde; und
b) einen EEG-Empfänger (55), der mit den Elektrodensensoren zum Empfangen von erzeugten EEG-Parametern verbunden ist,
wobei die Analyseeinheit (40) angepasst ist, die erzeugten EEG-Parameter zu empfangen, zu bestimmen, ob die erzeugten EEG-Parameter innerhalb eines erwarteten Bereichs von EEG-Parametern liegen, und das erste Warnsignal an die Rechtsdurchsetzungsentität oder die relevanten Behörden zu senden, wenn die erzeugten EEG-Parameter innerhalb des erwarteten Bereichs liegen und die erzeugten EEG-Parameter nach dem Detektieren des vorbestimmten Zielgeruchs auf die bekannte Reaktion des biologischen Sensors hinweisen.

4. System nach Anspruch 1, wobei der eine oder die mehreren zusätzlichen Signalerzeugungssensoren (22-24) zudem angepasst sind, eine entsprechende physikalische Reaktion des biologischen Sensors (14) während des Geruchsdetektiervorgangs zu erfassen, wobei die Analyseeinheit (40) betriebsfähig ist, Resultate des Geruchsdetektiervorgangs nicht zu berücksichtigen, wenn ein entsprechendes Signal, das durch den einen oder die mehreren zusätzlichen Sensoren erzeugt wird, nach dem Detektieren des vorbestimmten Zielgeruchs eine physikalische Reaktion darstellt, die mit der bekannten Reaktion des biologischen Sensors inkonsistent ist.

5. System nach Anspruch 1, wobei der eine oder die mehreren zusätzlichen Signalerzeugungssensoren ausgewählt sind aus der Gruppe von:
- einem Mikrofon, das am Körper des biologischen Sensors oder innerhalb der Kammer (12) installiert ist;
- einer Taste (21, 21A, 21B);
- einem EEG-Sensor (52);
- einem Herzfrequenzsensor (22) zum Bestimmen der Herzfrequenz des biologischen Sensors (14);
- einer Videokamera (24) zum Erfassen einer Körperbewegung des biologischen Sensors;
- einer Audioerfassungsvorrichtung zum Erfassen eines hörbaren Tons, der nach dem Detektieren des vorbestimmten Zielgeruchs durch den biologischen Sensor erzeugt wird; und
- einem Bewegungssensor (23) zum Erfassen einer Körperbewegung des biologischen Sensors.

6. System nach Anspruch 2, wobei der biologische Sensor ein Hund (14) ist, ferner umfassend eine Videokamera (24) zum Erfassen einer Körperbewegung des Hundes während des Geruchsdetektiervorgangs, wobei die Analyseeinheit (40) betriebsfähig ist, Resultate des Geruchsdetektiervorgangs nicht zu berücksichtigen, wenn eine durch die Videokamera erfasste Körperbewegung eine physikalische Reaktion darstellt, die mit der bekannten Reaktion des Hundes nach dem Detektieren des vorbestimmten Zielgeruchs inkonsistent ist.

7. System nach Anspruch 2, ferner umfassend Mittel (24) zum Erfassen der gemessenen physikalischen Reaktion des Annäherns an eine Schale (48), die sich innerhalb der Kammer (12) befindet, auf der Suche nach seiner Belohnung unmittelbar im Anschluss an die Detektion des vorbestimmten Geruchs und nachdem die Taste (21, 21A, 22B) gedrückt wurde, wobei die Analyseeinheit (40) betriebsfähig ist, Resultate des Geruchsdetektiervorgangs nicht zu berücksichtigen, wenn eine durch die Videokamera erfasste Körperbewegung eine physikalische Reaktion darstellt, die mit der bekannten Reaktion des Hundes nach dem Detektieren des vorbestimmten Zielgeruchs inkonsistent ist.

8. System nach Anspruch 1, wobei der biologische Sensor (14) ausgewählt ist aus der Gruppe von:
- einem Hund;
- Nagetieren;
- Schweinen;
- Vögeln;
- Säugetieren;
- beflügelten Kreaturen;
- Amphibien; und
Insekten.

9. System nach Anspruch 2, wobei der biologische Sensor (14) ein Säugetier ist, das trainiert ist, nach dem Detektieren des vorbestimmten Zielgeruchs die Taste (21, 21A, 22B) mit seiner Nase oder seinem Fuß zu drücken.

10. System nach Anspruch 2, wobei die Drückparameter ausgewählt sind aus der Gruppe bestehend aus:
- Kraft;
- Zeitdauer;
- Winkel; und
- Geschwindigkeit.

11. System nach Anspruch 1, ferner umfassend Mittel zum Erzeugen einer dominanten und/oder sekundären Ermutigung (26-28, 49), wenn der Zielgeruch identifiziert wird.

12. System nach Anspruch 11, wobei die dominante Ermutigung ausgewählt ist aus der Gruppe bestehend aus:
einer Lieblingsnahrung; und
einem Lieblingsspiel,
wobei die sekundäre Ermutigung ausgewählt ist aus der Gruppe bestehend aus:
- einem ausgeprägten Ton;
- einem hell klingenden Ton;
- einem klickenden Ton;
- einer Stimme, die von einem Lautsprecher emittiert wird, der in der Kammer installiert ist; und
- einem angenehmen Licht.

13. System nach Anspruch 11, wobei die Mittel zum Erzeugen einer dominanten Ermutigung, wenn ein Zielgeruch identifiziert wird, ein Dosierautomat (49) ist, und wobei optional der Dosierautomat (49) eine lebensmitteleinführbare Kammer (44) in Kommunikation mit der Kammer (12) und sich darüber befindend umfasst und ein lebensmittelabgabefähiges Element (45), das sich nach unten in ein Inneres der Kammer erstreckt und durch das die eingeführten Lebensmittel abgebbar sind.

14. System nach Anspruch 1, wobei die relevanten Behörden medizinische Behörden sind.

## Revendications

1. Système basé sur capteur biologique (10) destiné à détecter des odeurs, comprenant :
a) un compartiment (12) destiné à isoler complètement et enfermer un capteur biologique (14) situé dans celui-ci, ledit compartiment étant suffisamment grand pour permettre audit capteur biologique d'effectuer des mouvements d'animaux normaux ainsi qu'une opération de détection d'odeur ;
b) une admission d'air (17) dans ledit compartiment par laquelle de l'air chargé en odeurs peut être introduit dans un intérieur dudit compartiment ;
c) une sortie d'air (19) par laquelle ledit air chargé en odeurs peut être évacué dudit compartiment ;
d) au moins un capteur de génération de signal (21, 24, 25) destiné à mesurer des réactions physiques dudit capteur biologique en réponse à l'introduction à l'intérieur dudit compartiment dudit air chargé en odeurs ; et
e) une unité d'analyse (40) destinée à déterminer si les réactions physiques mesurées indiquent des réactions connues dudit capteur biologique lors de la détermination d'une odeur cible prédéterminée,
**caractérisé en ce que** :
ledit compartiment est suffisamment petit pour assurer l'absence d'espace dans ledit compartiment pour contenir un élément de traitement ou d'autres capteurs biologiques effectuant des opérations de détection d'odeur en plus dudit capteur biologique, et **en ce que** ledit capteur biologique ne pourra pas se déplacer ou errer dans celui-ci, pour conserver son énergie ;
ladite unité d'analyse est configurée pour transmettre automatiquement un premier signal d'alerte à une entité policière ou à des autorités officielles compétentes lorsque lesdites réactions mesurée se révèlent indiquer ladite réaction connue dudit capteur biologique lors de la détection de ladite odeur cible prédéterminée ;
ledit système comprenant en outre :
f) au moins un capteur de génération de signal supplémentaire (22 à 24) destiné à déterminer un niveau de vigilance du capteur biologique et le fait que le capteur biologique est ou non actuellement suffisamment performant pour continuer à effectuer lesdites opérations de détection d'odeurs, et
dans lequel ladite unité d'analyse (40) est également configurée pour recevoir un signal indiquant un niveau de vigilance depuis ledit au moins un capteur de génération de signal supplémentaire (22 à 24).

2. Système selon la revendication 1, comprenant en outre :
a) un bouton pouvant être actionné par un animal (21, 21A, 22B) qui est raccordé de manière mobile à une paroi dudit compartiment (12) est peut être actionné par ledit capteur biologique (14) lors de la détection du fait que ladite odeur introduite est l'odeur cible prédéterminée ; et
b) un capteur de force (31, 39) constituant ledit au moins un capteur de génération signal, qui est raccordé fonctionnellement audit bouton actionnable, destiné à détecter des paramètres de pression appliqués par ledit capteur biologique sur ledit bouton,
dans lequel ladite unité d'analyse (40) est adaptée pour déterminer le fait que lesdits paramètres de pression se trouvent ou non dans une plage prévue pour ledit capteur biologique et pour transmettre le premier signal d'alerte à l'entité policière ou aux autorités officielles compétentes lorsque lesdits paramètres de pression se trouvent dans ladite plage prévue et lesdits paramètres de pression indiquent ladite réaction connue dudit capteur biologique après la détection de ladite odeur cible prédéterminée.

3. Système selon la revendication 1, comprenant en outre :
a) un support (50) qui peut être porté sur la tête et/ou le corps dudit capteur biologique (14) et est muni d'une pluralité de capteurs de type électrode (52) constituant ledit au moins un capteur de génération de signal destinés à déterminer une activité électrique dans le cerveau au moyen d'un électroencéphalogramme (EEG), un profil d'onde prédéterminé indiquant un capteur biologique associé à un état mental de satisfaction lorsque ladite odeur cible prédéterminée a été détectée ; et
b) un récepteur d'EEG (55) connecté auxdits capteurs de type électrode destiné à recevoir des paramètres d'EEG générés,
dans lequel ladite unité d'analyse (40) est adaptée pour recevoir lesdits paramètres d'EEG générés, pour déterminer le fait que lesdits paramètres d'EEG générés se trouvent dans une plage prévue de paramètres d'EEG, et pour transmettre ledit premier signal d'alerte à l'entité policière ou aux autorités officielles compétentes lorsque lesdits paramètres d'EEG générés se trouvent dans ladite plage prévue et lesdits paramètres d'EEG générés indiquent ladite réaction connue dudit capteur biologique après la détection de ladite odeur cible prédéterminée.

4. Système selon la revendication 1, dans lequel l'au moins un capteur de génération de signal supplémentaire (22 à 24) est également adapté pour détecter une réaction physique correspondante du capteur biologique (14) pendant l'opération de détection d'odeur, l'unité d'analyse (40) pouvant fonctionner pour ne pas tenir compte de résultats de l'opération de détection d'odeur lorsqu'un signal correspondant généré par ledit au moins un capteur supplémentaire est représentatif d'une réaction physique qui n'est pas cohérente avec la réaction connue du capteur biologique après la détection de l'odeur cible prédéterminée.

5. Système selon la revendication 1, dans lequel l'au moins un capteur de génération de signal supplémentaire est choisi dans le groupe constitué par :
- un microphone installé sur le corps du capteur biologique ou dans le compartiment (12) ;
- un bouton-poussoir (21, 21A, 21B) ;
- un capteur d'EEG (52) ;
- un capteur de rythme cardiaque (22) destiné à déterminer le rythme cardiaque du capteur biologique (14) ;
- une caméra vidéo (24) destinée à capturer un mouvement corporel du capteur biologique ;
- un dispositif de capture audio destiné à capturer un son audible produit par le capteur biologique lors de la détection de l'odeur cible prédéterminée ; et
- un capteur de mouvement (23) destiné à capturer un mouvement corporel du capteur biologique.

6. Système selon la revendication 2, dans lequel le capteur biologique est un chien (14), comprenant en outre une caméra vidéo (24) destinée à capturer un mouvement corporel du chien pendant l'opération de détection d'erreur, dans lequel l'unité d'analyse (40) peut fonctionner pour ne pas tenir compte de résultats de l'opération de détection lorsqu'un mouvement corporel capturé par ladite caméra vidéo est représentatif d'une réaction physique qui n'est pas cohérente avec la réaction connue du chien après la détection de l'odeur cible prédéterminée.

7. Système selon la revendication 2, comprenant en outre un moyen (24) destiné à capturer la réaction physique mesurée liée au fait de s'approcher d'un bol (48) situé dans le compartiment (12) à la recherche de sa récompense immédiatement après la détection de l'odeur prédéterminée et après l'actionnement du bouton (21, 21A, 22B), dans lequel l'unité d'analyse (40) peut fonctionner pour ne pas tenir compte de résultats de l'opération de détection d'odeur lorsqu'un mouvement corporel capturé par ladite caméra vidéo est représentatif d'une réaction physique qui n'est pas cohérente avec la réaction connue du chien après la détection de l'odeur cible prédéterminée.

8. Système selon la revendication 1, dans lequel le capteur biologique (14) est choisi dans le groupe constitué par :
- un chien ;
- des rongeurs ;
- des porcs ;
- des oiseaux ;
- des mammifères ;
- des créatures ailées ;
- des amphibiens ; et
- des insectes.

9. Système selon la revendication 2, dans lequel le capteur biologique (14) est un mammifère, entraîné pour actionner le bouton (21, 21A, 22B) avec son nez ou sa patte lors de la détection de l'odeur cible prédéterminée.

10. Système selon la revendication 2, dans lequel les paramètres de pression sont choisis dans le groupe constitué par :
- la force ;
- la durée ;
- l'angle ; et
- la vitesse.

11. Système selon la revendication 1, comprenant en outre un moyen destiné à générer un encouragement dominant et/ou secondaire (26 à 28, 29) lorsque l'odeur cible est identifiée.

12. Système selon la revendication 11, dans lequel l'encouragement dominant est choisi dans le groupe constitué par :
- un aliment favori ; et
- un jeu favori,
dans lequel l'encouragement secondaire est choisi dans le groupe constitué par :
- un son distinctif ;
- un son aigu ;
- un son de claquement ;
- une voix émise par un haut-parleur qui est installé dans le compartiment ; et
- une lumière agréable.

13. Système selon la revendication 11, dans lequel le moyen destiné à générer un encouragement dominant lorsqu'une odeur cible est identifiée est un distributeur automatique (49), et éventuellement
dans lequel le distributeur automatique (49) comprend un chambre d'introduction d'aliments (44) en communication avec le compartiment (12) et située au-dessus de celui-ci, et un élément de décharge d'aliments (45) s'étendant vers le bas dans un intérieur du compartiment et à travers lequel lesdits aliments introduits peuvent être distribués.

14. Système selon la revendication 1, dans lequel les autorités officielles compétentes sont des autorités médicales.
